(19) Europäisches Patentamt
European Patent Office
Office européen des brevets

(11) Veröffentlichungsnummer: **0 046 557**
**B1**

(12) # EUROPÄISCHE PATENTSCHRIFT

(45) Veröffentlichungstag der Patentschrift: **22.02.84**

(51) Int. Cl.³: **C 07 C 145/02**, A 01 N 47/34

(21) Anmeldenummer: **81106374.2**

(22) Anmeldetag: **17.08.81**

(54) **N-Sulfenylierte Harnstoffe, Verfahren zu ihrer Herstellung, diese enthaltende fungizide Mittel und ihre Verwendung.**

(30) Priorität: **27.08.80 DE 3032327**

(43) Veröffentlichungstag der Anmeldung: **03.03.82 Patentblatt 82/9**

(45) Bekanntmachung des Hinweises auf die Patenterteilung: **22.02.84 Patentblatt 84/8**

(84) Benannte Vertragsstaaten: **AT BE CH DE FR GB IT LI NL SE**

(56) Entgegenhaltungen: **US - A - 3 496 208**

(73) Patentinhaber: **BAYER AG, Zentralbereich Patente, Marken und Lizenzen, D-5090 Leverkusen 1, Bayerwerk (DE)**

(72) Erfinder: **Kühle, Engelbert, Dr., von-Bodelschwingh-Strasse 42, D-5060 Bergisch Gladbach 2 (DE)**
Erfinder: **Brandes, Wilhelm, Dr., Eichendorffstrasse 3, D-5623 Leichlingen (DE)**
Erfinder: **Frohberger, Paul-Ernst, Dr., Willi-Baumeister-Strasse 5, D-5090 Leverkusen 1 (DE)**

**EP 0 046 557 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

N-Sulfenylierte Harnstoffe, Verfahren zu ihrer Herstellung, diese enthaltende fungizide Mittel und ihre Verwendung

Die vorliegende Erfindung betrifft neue N-sulfenylierte Harnstoffe, ein Verfahren zu ihrer Herstellung, fungizide Mittel die diese Verbindungen enthalten, sowie ihre Verwendung.

Es ist bereits bekannt geworden, daß N-Trihalogen-methansulfenyl-dicarbonimide eine fungizide Aktivität besitzen. So wird z. B. N-Trichlormethansulfenyl-tetrahydrophthalimid seit mehreren Jahren als Blattfungizid in verschiedenen Kulturen eingesetzt (vgl. US-PS 2 553 770). Bezüglich der Wirksamkeit befriedigt dieses Produkt nicht immer. Weiterhin sind auch N-sulfenylierte Carbamoylverbindungen mit einem Trihalogenmethansulfenyl-Rest fungizid wirksam; auch bei dieser Verbindungsklasse ist die fungizide Wirkung nicht immer ausreichend (vgl. hierzu DE-OS 2 354 492). Weiterhin sind N-Trichlormethylthiocarbanilide, wie das N-Trichlormethylthio-4'-chlorcarbanilide als Bakterizide bekannt (vgl. hierzu US-PS 3 496 208).

Es wurden die neuen N-sulfenylierten Harnstoffe der allgemeinen Formel (I) gefunden:

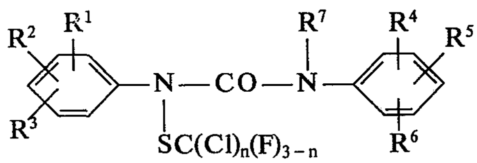

(I)

in welcher

$R^1-R^6$ gleich oder verschieden sein können und für Wasserstoff, Halogen, Nitro, Cyano, Alkyl, Alkoxy, Alkylmercapto, Trihalogenmethyl, Trihalogenmethoxi und/oder Trihalogenmethylmercapto stehen

$R^7$ für Wasserstoff oder Alkyl steht und

n die Zahlen 0, 1 und 2 bedeuten.

Weiterhin wurde gefunden, daß man die N-sulfenylierten Harnstoffe der Formel (I)

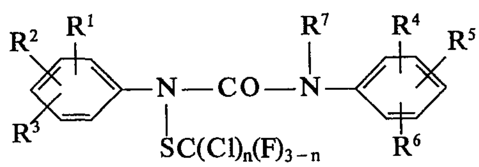

(I)

in welcher

$R^1-R^6$ gleich oder verschieden sein können und für Wasserstoff, Halogen, Nitro, Cyano, Alkyl, Alkoxy, Alkylmercapto, Trihalogenmethyl, Trihalogenmethoxi und/oder Trihalogenmethylmercapto stehen

$R^7$ für Wasserstoff oder Alkyl steht und

n die Zahlen 0, 1 und 2 bedeuten,

erhält, wenn man N-sulfenylierte Carbamidsäurefluoride der Formel (II)

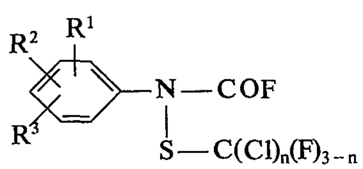

(II)

in welcher

$R^1-R^3$
und n die oben angegebene Bedeutung haben,

mit einem Anilin der Formel (III)

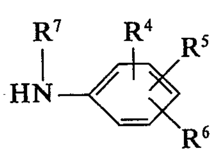

(III)

in welcher

$R^4-R^7$ die oben angegebene Bedeutung haben,

in Gegenwart eines Verdünnungsmittels und eines säurebindenden Mittels umsetzt.

Die erfindungsgemäßen Verbindungen weisen eine ausgezeichnete fungizide Wirkung auf. Es ist als überraschend zu bezeichnen, daß die erfindungsgemäßen Verbindungen eine höhere fungizide Wirkung aufweisen als die aus dem Stand der Technik bekannten nächstliegenden Verbindungen.

Bevorzugt sind Verbindungen der Formel (I), in welcher $R^1-R^6$ für gleiche oder verschiedene Reste aus der Gruppe Wasserstoff, Halogen, Nitro, Cyano, $C_{1-4}$-Alkyl, $C_{1-4}$-Alkoxy, $C_{1-4}$-Alkylenmercapto, Trihalogenmethyl, Trihalogenmethoxy, Trihalogenmethylmercapto stehen.

Besonders bevorzugt sind Verbindungen der Formel (I), in welcher $R^1-R^6$ für Wasserstoff, Chlor, Trifluormethyl oder Methoxy stehen.

Der Reaktionsverlauf läßt sich bei Verwendung von N-(Fluordichlormethylsulfenyl)-4-chlorphenylcarbamidsäurefluorid und 3,4-Dichloranilin als Ausgangsmaterialien durch folgendes Formelbild wiedergeben:

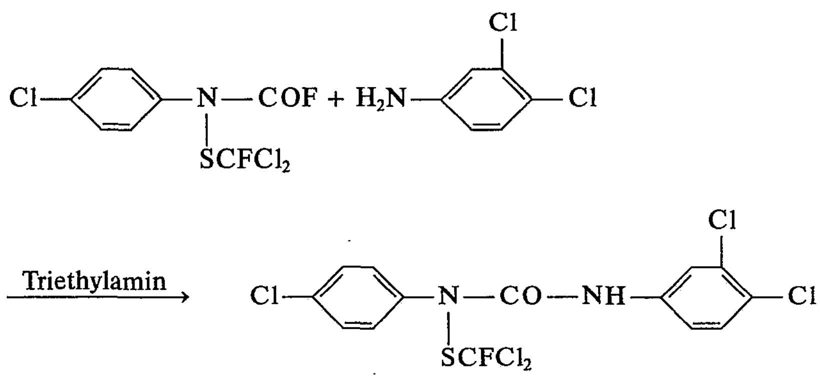


Die als Ausgangsmaterialien zu verwendenden N-sulfenylierten Carbamidsäurefluoride sind durch die Formel (II) eindeutig definiert. In dieser Formel steht n vorzugsweise für die Zahl 2. Die Reste $R^1-R^3$ bedeuten vorzugsweise Wasserstoff, Chlor, Nitro, Cyano, Niederalkyl, Niederalkoxi, Niederalkylmercapto, Trifluormethyl, Difluorchlormethyl, Trifluormethoxi und/oder Trifluormethylmercapto. Diese Verbindungen sind bekannt (De-AS 1 297 095) oder können in bekannter Weise aus Acrylcarbamidsäurefluoriden und Trihalogenmethansulfenylchlorid in Gegenwart eines säurebindenden Mittels hergestellt werden.

Als Beispiele seien genannt: N-(Fluordichlormethylsulfenyl)-phenylcarbamidsäurefluorid, die entsprechenden N-(Fluordichlormethylsulfenyl)-arylcarbamidsäurefluoride des 2-Chloranilins, 3,4-Dichloranilins, 3-Nitroanilins, 4-Toluidins, 4-Isopropylanilins, 3-Chlor-4-methoxianilins, 2-Chlor-4-methylmercaptoanilins, 2-Chlor-4-trifluormethylanilins, 4-Difluorchlormethylanilins, 3-Chlor-4-trifluormethoxianilins und 3-Trifluormethylmercaptoanilins.

Die zur Umsetzung weiterhin benötigten Aniline sind durch die Formel (III) definiert. In dieser Formel stehen die Reste $R^4-R^6$ vorzugsweise für Wasserstoff, Chlor, Nitro, Cyano, Niederalkyl, Niederalkoxi, Niederalkylmercapto, Trifluormethyl, Trifluormethoxi, Difluorchlormethoxi, Trifluormethylmercapto, Difluorchlormethylmercapto. $R^7$ steht vorzugsweise für Wasserstoff oder Niederalkyl, insbesondere Methyl.

Die Aniline der Formel (III) sind bekannt oder können in an sich bekannter Weise durch Reduktion der entsprechenden Nitroverbindungen hergestellt werden.

Die Herstellung der erfindungsgemäßen Verbindungen erfolgt bevorzugt in organischen Lösungsmitteln. Hierzu gehören vorzugsweise Ether wie Diethylether, Tetrahydrofuran und Dioxan; Kohlenwasserstoffe wie Toluol; Chlorkohlenwasserstoffe wie Chloroform und Chlorbenzol. Zur Bindung des bei der Reaktion entstehenden Fluorwasserstoffs setzt man dem Reaktionsgemisch ein Säurebindemittel zu. Bevorzugt verwendet man ein tertiäres Amin wie Pyridin oder Triethylamin oder organische Basen wie Alkali- oder Erdalkalihydroxide oder Alkalicarbonate. Man kann auch das zur Umsetzung verwendete Anilin in doppeltem Überschuß einsetzen.

Die Reaktionstemperaturen können in einem größeren Bereich variiert werden, im allgemeinen arbeitet man zwischen 0 und 100° C, vorzugsweise bei 20—50° C.

Im allgemeinen arbeitet man bei Normaldruck. Die Ausgangsverbindungen werden in molarem Verhältnis zueinander eingesetzt. Ein Überschuß der einen oder anderen Komponente bringt keine wesentlichen Vorteile. Die Aufarbeitung nach erfolgter Reaktion erfolgt in üblicher Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Eine besonders gute Wirksamkeit entfalten sie gegen parasitäre Pilze auf oberirdischen Pflanzenteilen, wie z. B. gegen Phytophthora-Arten und gegen den Erreger des Apfelschorfs (Fusicladium dendriticum). Sie zeigen ferner eine Wirksamkeit gegen Pyricularia oryzae.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benuzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol oder Alkylnaphthalino, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreise, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

## Herstellungsbeispiele

### Beispiel 1

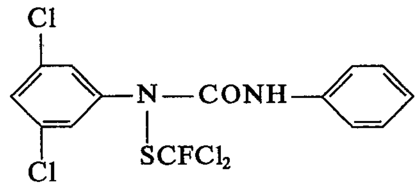

10,2 g (0,03 Mol) N-(Fluordichlormethansulfenyl)-3,5-dichlorphenylcarbamidsäurefluorid werden in 100 ml Toluol gelöst. Dazu tropft man eine Lösung von 6 g (0,07 Mol) Anilin in 20 ml Toluol. Hierbei steigt die Temperatur leicht an. Man rührt eine Stunde lang bei 50—60°, säuert nach Zugabe von Wasser mit Salzsäure an und engt die Toluollösung ein. Als Rückstand erhält man 12 g des N-(Fluordichlormethylsulfenyl)-N-3,5-dichlorphenyl-N'-phenylharnstoffs vom Schmp. 100—102°.

In analoger Weise können folgende erfindungsgemäße Verbindungen hergestellt werden:

| | | Schmelzpunkt °C |
|---|---|---|
| 2. | 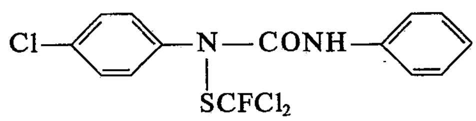 | 104–105 |
| 3. | 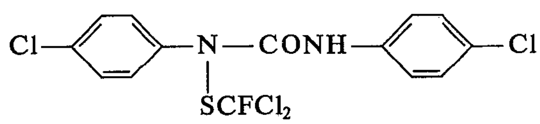 | 125–126 |
| 4. | 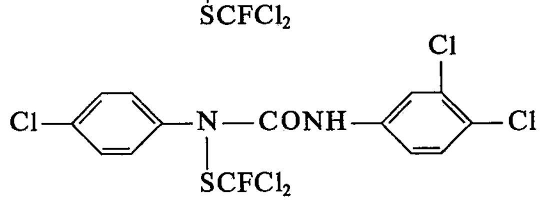 | 158–162 |
| 5. | 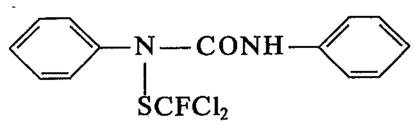 | 109–111 |
| 6. | 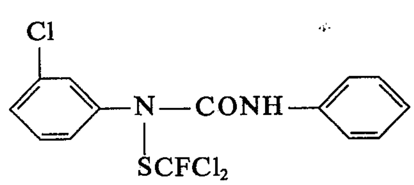 | 97– 98 |
| 7. | 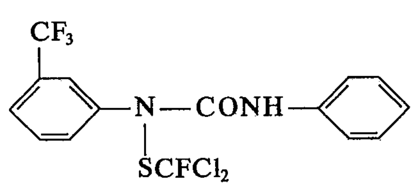 | 100–101 |
| 8. | 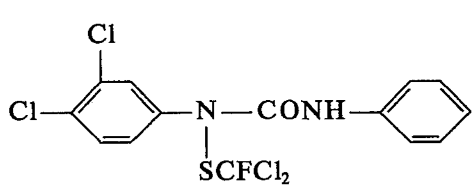 | 123–125 |

Fortsetzung

| | | Schmelzpunkt °C |
|---|---|---|
| 9. | 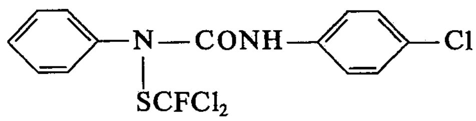 | 80- 85 |
| 10. | 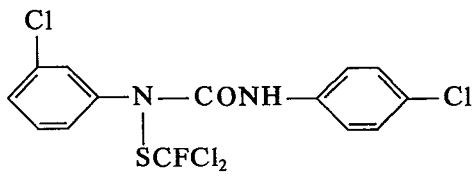 | 83- 88 |
| 11. | 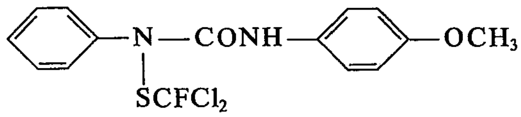 | 94- 96 |
| 12. | 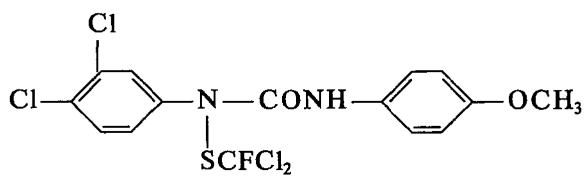 | 128-131 |
| 13. | 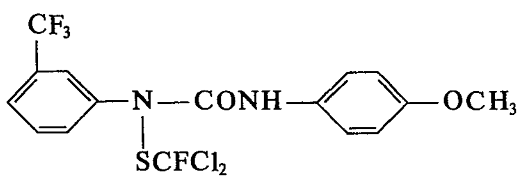 | 92 |
| 14. | 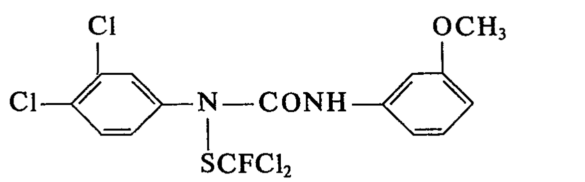 | 127-132 |
| 15. | 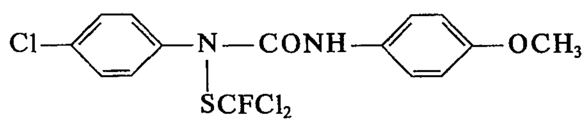 | 108 |

Herstellung der Ausgangsverbindungen:

Beispiel I

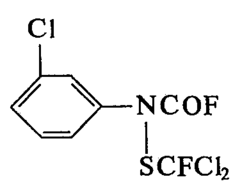

In eine Lösung aus 212 g 3-Chlorphenylcarbamidsäurefluorid (aus 3-Chlorphenylisocyanat und wasserfreier Flußsäure) und 217 g Fluordichlormethansulfenylchlorid in 600 ml Chlorbenzol tropft man 185 ml Triethylamin. Durch Eiswasserkühlung hält man die Temperatur unterhalb von 30°C. Man rührt

eine Zeitlang, versetzt mit Wasser und engt die organische Lösung nach dem Trocknen i. Vak. ein. Durch Destillation erhält man 304 g des N-(Fluordichlormethylsulfenyl)-3-chlorphenylcarbamidsäurefluorid vom $Sdp._{0,05}$ 85–90°. Lt. GC 96,4%ig.

Analog erhält man

II 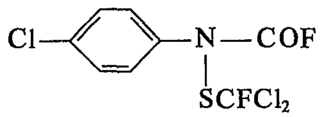 $Sdp._{0,05}$ 94–100°

III 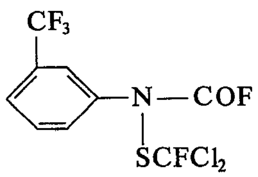 $Sdp._{0,05}$ 82°

IV 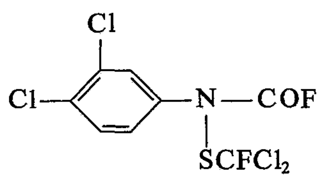 $Sdp._{0,12}$ 125°

V 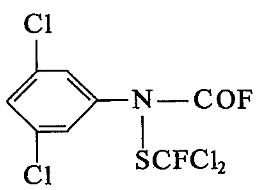 $Sdp._{0,15}$ 108–110°

Beispiel A
Phytophthora-Test (Tomate)/protektiv

Lösungsmittel: 4,7 Gewichtsteile Aceton
Emulgator: 0,3 Gewichtsteile Alkylarylpolyglykoläther

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit bei ca. 20° C aufgestellt.

4 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele: 1, 2, 3, 5, 6, 7, 8, 9, 10, 11, 12, 13.

Beispiel
Fusicladium-Test (Apfel)/protektiv

Lösungsmittel: 4,7 Gewichtsteile Aceton
Emulgator: 0,3 Gewichtsteile Alkyl-aryl-polyglykoläther
Wasser: 95 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20° C und einer relativen

Luftfeuchtigkeit von 70% im Gewächshaus. Anschließend werden sie mit einer wäßrigen Konidiensuspension des Apfelschorferregers (Fusicladium dendriticum) inokuliert und 18 Stunden lang in einer Feuchtkammer bei 18 bis 20°C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Dabei zeigen z. B. die folgenden Verbindungen Überlegenheit in der Wirksamkeit gegenüber dem Stand der Technik: 1, 2, 4, 6, 7, 9, 10, 11, 14.

Beispiel

Fusarium nivale-Test (Roggen)/Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Roggen sät man mit 2×100 Korn 1 cm tief in eine Standarderde und kultiviert ihn im Gewächshaus bei einer Temperatur von ca. 10°C und einer relativen Luftfeuchtigkeit von ca. 95% in Saatkästen, die täglich 15 Stunden dem Licht ausgesetzt werden.

Ca. 3 Wochen nach der Aussaat erfolgt die Auswertung der Pflanzen auf Symptome des Schneeschimmels.

Eine deutliche Überlegenheit in der Wirksamkeit gegenüber dem Stand der Technik zeigen bei diesem Test z. B. die Verbindungen gemäß folgender Herstellungsbeispiele: 5, 9, 10.

**Patentansprüche**

1. N-sulfenylierte Harnstoffe der Formel (I)

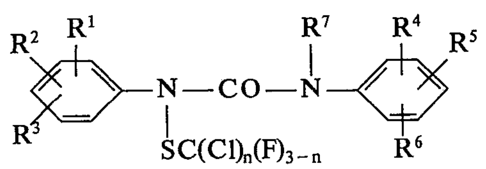

(I)

in welcher

$R^1$—$R^6$ gleich oder verschieden sein können und für Wasserstoff, Halogen, Nitro, Cyano, Alkyl, Alkoxy, Alkylmercapto, Trihalogenmethyl, Trihalogenmethoxi und/oder Trihalogenmethylmercapto stehen,

$R^7$ für Wasserstoff oder Alkyl steht und

n die Zahlen 0, 1 und 2 bedeuten.

2. Verfahren zur Herstellung der N-sulfenylierten Harnstoffe der Formel (I)

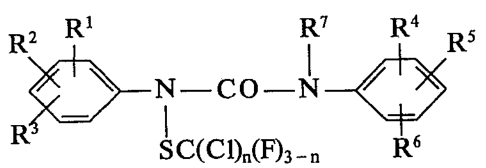

(I)

in welcher

$R^1$—$R^6$ gleich oder verschieden sein können und für Wasserstoff, Halogen, Nitro, Cyano, Alkyl, Alkoxy, Alkylmercapto, Trihalogenmethyl-, Trihalogenmethoxi und/oder Trihalogenmethylmercapto stehen,

$R^7$ für Wasserstoff oder Alkyl steht und

n die Zahlen 0, 1 und 2 bedeuten,

dadurch gekennzeichnet, daß man N-sulfenylierte Carbamidsäurefluoride der Formel (II)

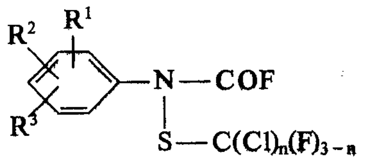

(II)

in welcher

$R^1-R^3$
und n die oben angegebene Bedeutung haben,

mit einem Anilin der Formel (III)

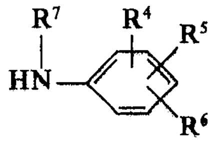

(III)

in welcher

$R^4-R^7$ die oben angegebene Bedeutung haben,

in Gegenwart eines Verdünnungsmittels und eines säurebindenden Mittels umsetzt.

3. Verbindungen der Formel (I) in welcher

$R^1-R^6$ für gleiche oder verschiedene Reste aus der Gruppe Wasserstoff, Halogen, Nitro, Cyano, $C_{1-4}$-Alkyl, $C_{1-4}$-Alkoxy, $C_{1-4}$-Alkylmercapto, Trihalogenmethyl, Trihalogenmethoxy, Trihalogenmethylmercapto stehen und
$R^7$ und n die oben angegebene Bedeutung haben.

4. Verbindungen der Formel (I) in welcher

$R^1-R^6$ für Wasserstoff, Chlor, Trifluormethyl oder Methoxy stehen und
$R^7$ und n die oben angegebene Bedeutung haben.

5. Fungizide Mittel, gekennzeichnet durch einen Gehalt an mindestens einem N-sulfenylierten Harnstoff der Formel (I).

6. Verwendung von N-sulfenylierten Harnstoffen der Formel (I) zur Bekämpfung von planzenpathogenen Pilzen.

7. Verfahren zur Bekämpfung von pflanzenpathogenen Pilzen, dadurch gekennzeichnet, daß man N-sulfenylierte Harnstoffe der Formel (I) auf pflanzenpathogene Pilze und/oder ihren Lebensraum einwirken läßt.

**Claims**

1. N-Sulphenylated ureas of the formula (I)

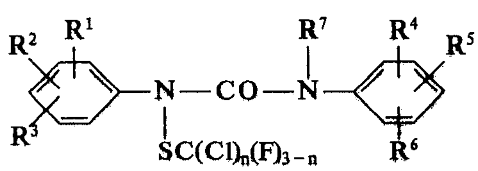

(I)

in which

$R^1-R^6$ can be identical or different and represent hydrogen, halogen, nitro, cyano, alkyl, alkoxy, alkylmercapto, trihalogenomethyl, trihalogenomethoxy and/or trihalogenomethylmercapto,
$R^7$ represents hydrogen or alkyl and
n denotes the number 0, 1 or 2.

2. Process for the preparation of the N-sulphenylated ureas of the formula (I)

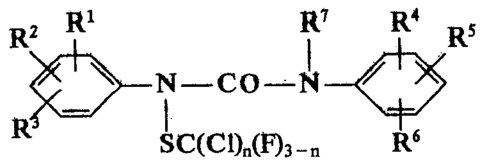

(I)

in which

$R^1-R^6$ can be identical or different and represent hydrogen, halogen, nitro, cyano, alkyl, alkoxy, alkylmercapto, trihalogenomethyl, trihalogenomethoxy and/or trihalogenomethylmercapto,
$R^7$ represents hydrogen or alkyl and
n denotes the number 0, 1 or 2,

characterised in that N-sulphenylated carbamic acid fluorides of the formula (II)

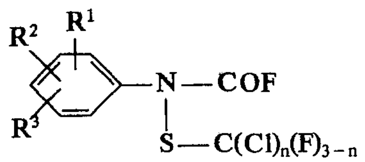

(II)

in which

$R^1-R^3$
and n have the abovementioned meaning,

are reacted with an aniline of the formula (III)

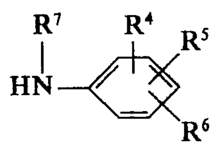

(III)

in which

$R^4-R^7$ have the abovementioned meaning,

in the presence of a diluent and an acid-binding agent.

3. Compounds of the formula (I) in which

$R^1-R^6$ represent identical or different radicals from the group comprising hydrogen, halogen, nitro, cyano, $C_{1-4}$-alkyl, $C_{1-4}$-alkoxy, $C_{1-4}$-alkylmercapto, trihalogenomethyl, trihalogenomethoxy or trihalogenomethylmercapto and
$R^7$ and n have the abovementioned meaning.

4. Compounds of the formula (I) in which

$R^1-R^6$ represent hydrogen, chlorine, trifluoromethyl or methoxy and
$R^7$ and n have the abovementioned meaning.

5. Fungicidal agents, characterised in that they contain at least one N-sulphenylated urea of the formula (I).

6. Use of N-sulphenylated ureas of the formula (I) for combating phytopathogenic fungi.

7. Process for combating phytopathogenic fungi, characterised in that N-sulphenylated ureas of the formula (I) are allowed to act on phytopathogenic fungi and/or their environment.

**Revendications**

1. Urées N-sulfénylées de formule I

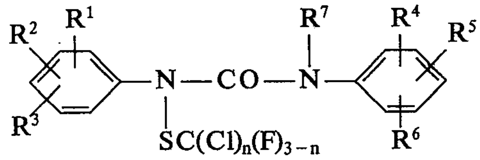

(I)

dans laquelle

$R^1$ à $R^6$ ayant les significations identiques ou différentes, représentent chacun l'hydrogène, un halogène, un groupe nitro, cyano, alkyle, alcoxy, alkylmercapto, trihalogénométhyle, trihalogénométhoxy et/ou trihalogénométhylmercapto,

$R^7$ représente l'hydrogène ou un groupe alkyle, et

n est égal à 0, 1 ou 2.

2. Procédé de préparation des urées N-sulfénylées de formule I

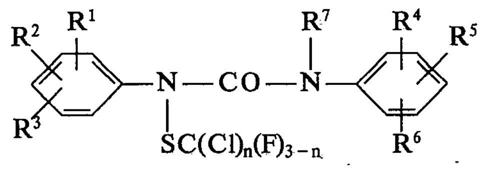

(I)

dans laquelle

$R^1$ à $R^6$ ayant des significations identiques ou différentes, représentant chacun l'hydrogène, un halogène, un groupe nitro, cyano, alkyle, alcoxy, alkylmercapto, trihalogénométhyle, trihalogénométhoxy et/ou trihalogénométhylmercapto,

$R^7$ représente l'hydrogène ou un groupe alkyle, et

n est égal à 0, 1 ou 2,

caractérisé en ce que l'on fait réagir des fluorures d'acides carbamiques N-sulfénylés de formule II

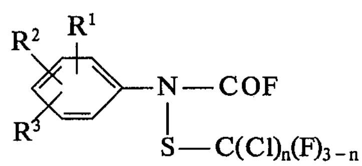

(II)

dans laquelle

$R^1$ à $R^3$
et n ont les significations indiquées ci-dessus,

avec une aniline de formule III

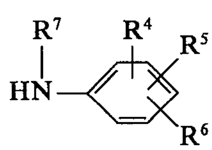

(III)

dans laquelle

$R^4$ à $R^7$ ont les significations indiquées ci-dessus,

en présence d'un diluant et d'un agent fixant les acides.

3. Composé de formule I dans laquelle

$R^1$ à $R^6$ représentent des restes identiques ou différents pris dans le groupe de l'hydrogène, des halogènes, des groupes nitro, cyano, alkyle en $C_1$—$C_4$, alcoxy en $C_1$—$C_4$, alkylmercapto en

$C_1-C_4$, trihalogénométhyle, trihalogénométhoxy, trihalogénométhylmercapto, et
$R^7$ et n ont les significations indiquées ci-dessus.

4. Composés de formule I dans laquelle

$R^1$ à $R^6$ représentent l'hydrogène, le chlore, des groupes trifluorométhyle ou méthoxy, et
$R^7$ et n ont les significations indiquées ci-dessus.

5. Produits fongicides, caractérisés en ce qu'ils contiennent au moins une urée N-sulfénylée de formule I.

6. Utilisation des urées N-sulfénylées de formule I dans la lutte contre les mycètes phytopathogènes.

7. Procédé pour combattre les mycètes phytopathogènes, caractérisé en ce que l'on fait agir des urées N-sulfénylées de formule I sur les mycètes phytopathogènes et/ou leur habitat.